(19) **Europäisches Patentamt**

**European Patent Office**

**Office européen des brevets**

(11) **EP 0 734 700 A2**

(12) **EUROPÄISCHE PATENTANMELDUNG**

(43) Veröffentlichungstag:
02.10.1996 Patentblatt 1996/40

(51) Int. Cl.$^6$: **A61F 2/30**, A61F 2/38

(21) Anmeldenummer: 96108124.7

(22) Anmeldetag: 08.12.1992

(84) Benannte Vertragsstaaten:
AT BE CH DE DK ES FR GB GR IE IT LI LU MC NL PT SE

(30) Priorität: 11.12.1991 DE 4140837
31.01.1992 DE 4202717

(62) Anmeldenummer der früheren Anmeldung nach Art. 76 EPÜ: 92924687.4

(71) Anmelder:
• **Kubein-Meesenburg, Dietmar, Prof. Dr.**
  **D-37547 Kreiensen (DE)**
• **NÄGERL, Hans Dr.**
  **D-37130 Gleichen (DE)**
• **Theusner, Joachim, Dr.**
  **D-80539 München (DE)**

(72) Erfinder:
• **Kubein-Meesenburg, Dietmar, Prof. Dr.**
  **37547 Kreiensen (DE)**
• **Nägerl, Hans, Prof. Dr.**
  **37130 Gleichen (DE)**

(74) Vertreter: **Patentanwälte**
**Dr. Solf & Zapf**
**Postfach 13 01 13**
**42028 Wuppertal (DE)**

Bemerkungen:
This application was filed on 22 - 05 - 1996 as a divisional application to the application mentioned under INID code 62.

(54) **Künstliches Gelenk**

(57)     Die vorliegende Erfindung betrifft ein künstliches Gelenk als Endoprothese für das menschliche Kniegelenk. Die vorliegende Erfindung besteht aus einem ersten, aus einem ersten Gelenkkopf und einer ersten Gelenkpfanne gebildeten Gelenk und einem zweiten, aus einem zweiten Gelenkkopf und einer zweiten Gelenkpfanne gebildeten Gelenk, die zueinander derart parallel angeordnet sind, daß die jeweiligen Rotationsachsen der beiden Gelenke parallel zueinander verlaufen, und daß das zweite Gelenk gegenüber dem ersten Gelenk in der Längsebene gesehen zurückversetzt ist sowie die Gelenkköpfe untereinander und die Gelenkpfannen untereinander starr verbindbar sind. Hierbei weisen die Gelenkteile Funktionsflächen auf, deren Krümmungsverhältnisse in ihrer Längsebene und in der hierzu um 90° versetzten Querebene entweder konvex-konvex und/oder konvex-konkav sind, und die Lenkgeometrie der Funktionsflächen zueinander in jeder der beiden Ebenen durch eine Gelenkkette mit zwei Gelenkachsen bestimmt ist, die durch die Rotationszentren ($M_1$, $M_2$; $M_8$, $M_9$) der Funktionsflächen mit den Radien ($R_1$, $R_2$; $R_8$, $R_9$) der jeweilig zugehörigen Schnittkonturen verlaufen. Hierbei entsprechen eine femurseitige Verbindung (F) der Mittelpunkte ($M_1$, $M_8$) einem Gestell und eine tibiaseitige Verbindung (T) der Mittelpunkte ($M_2$, $M_9$) einer Koppel einer die vier Rotationsachsen aufweisenden Viergelenkkette.

*FIG 5*

**Beschreibung**

Die vorliegende Erfindung betrifft ein künstliches Gelenk als eine Endoprothese für das menschliche Kniegelenk, bestehend aus mindestens zwei Zueinander sich bewegenden Gelenkteilen mit gekrümmten Gelenkflächen.

Aus der deutschen Patentanmeldung P 39 08 958.4 ist bereits ein künstliches Gelenk bekannt, zum Ersatz insbesondere von menschlichen Gelenken, bestehend aus mindestens zwei Gelenkteilen mit zueinander sich bewegenden sphärischen Funktionsflächen. Die Krümmungsverhältnisse der eine kreisförmige Schnittkontur aufweisenden Funktionsflächen sind zueinander konvex-konvex, konvex-konkav oder konkav-konkav, und die Gelenkgeometrie ist durch eine Gelenkkette mit zwei Gelenkachsen (dimere Gelenkkette) bestimmt, die durch die Rotationszentren der Funktiosflächen verlaufen. Hierbei sind die Gelenkflächen kugelförmig ausgebildet, so daß eine Gelenkbewegung mit fünf Freiheitsgraden möglich ist.

Es hat sich jedoch gezeigt, daß derartige Gelenke nicht geeignet sind, um spezielle Gelenkfunktionen des menschlichen Kniegelenks nachzubilden.

Der Erfindung liegt die Aufgabe zugrunde, ein künstliches Gelenk zu schaffen, das eine Bewegungsfreiheit nur in einer Gelenkebene besitzt und das gleichzeitig eine hohe mechanische Stabilität mit einer großen Variationsbreite zur Anpassung an individuelle Gegebenheiten aufweist.

Erfindungsgemäß wird dies durch ein künstliches Gelenk als Endoprothese für das menschliche Kniegelenk erreicht, bestehend aus einem ersten, aus einem ersten Gelenkkopf und einer ersten Gelenkpfanne gebildeten Gelenk und einem zweiten, aus einem zweiten Gelenkkopf und einer zweiten Gelenkpfanne gebildeten Gelenk, die zueinander derart parallel angeordnet sind, daß die jeweiligen Rotationsachsen der beiden Gelenke parallel zueinander verlaufen, und daß das zweite Gelenk gegenüber dem ersten Gelenk in der Längsebene gesehen zurückversetzt ist sowie die Gelenkköpfe untereinander und die Gelenkpfannen untereinander starr verbindbar sind, wobei die Gelenkteile Funktionsflächen aufweisen, deren Krümmungsverhältnisse in ihrer Längsebene und in der hierzu um 90° versetzten Querebene entweder konvex-konvex und/oder konvex-konkav sind, und die Gelenkgeometrie der Funktionsflächen zueinander in jeder der beiden Ebenen durch eine Gelenkkette mit zwei Gelenkachsen bestimmt ist, die durch die Rotationszentren der Funktionsflächen mit den Radien der jeweils zugehörigen Schnittkonturen verlaufen, wobei eine femurseitige Verbindung F der Mittelpunkte der Gelenkköpfe einem Gestell und eine tibiaseitige Verbindung T der Mittelpunkte der Gelenkpfannen einer Koppel einer die vier Rotationsachsen aufweisenden Viergelenkkette entsprechen.

Vorteilhafte Eigenschaften und Ausführungen sind in den Unteransprüchen enthalten. Anhand der in den beiliegenden Zeichnungen enthaltenen Ausführungsbeispiele wird die Erfindung näher erläutert. Es zeigen:

Fig. 1      einen Schnitt in der sagittalen Ebene (Längsebene) durch eine erste Ausführungsform eines Gelenks mit Druckverteilungskörper,

Fig. 2      einen Schnitt durch das Gelenkteil gemäß Fig. 1, jedoch in der frontalen Ebene, d.h. der um 90° versetzten Querebene zu Fig. 1,

Fig. 3      einen Schnitt in der sagittalen Ebene (Längsebene) durch eine weitere Ausführungsform eines Gelenkes mit Druckverteilungskörper,

Fig. 4      einen Schnitt durch das Gelenk gemäß Fig. 3, jedoch in der frontalen Ebene, d.h. in einer um 90° gedrehten Querebene zu Fig. 3,

Fig. 5      eine Anordnung einer erfindungsgemäßen Endoprothese für das rechte menschliche Knie im sagittalen Schnitt (Längsschnitt, seitliche Ansicht).

Fig.6 u 7      Ansichten gemäß den Fig. 1 und 2 durch ein Gelenkteil ohne Druckverteilungskörper,

Fig.8 u 9      Ansichten gemäß den Fig. 3 und 4 durch ein Gelenkteil ohne Druckverteilungskörper,

Fig. 10      eine Anordnung einer erfindungsgemäßen Endoprothese für das rechte menschliche Knie im sagittalen Schnitt in einer weiteren Ausführungsform.

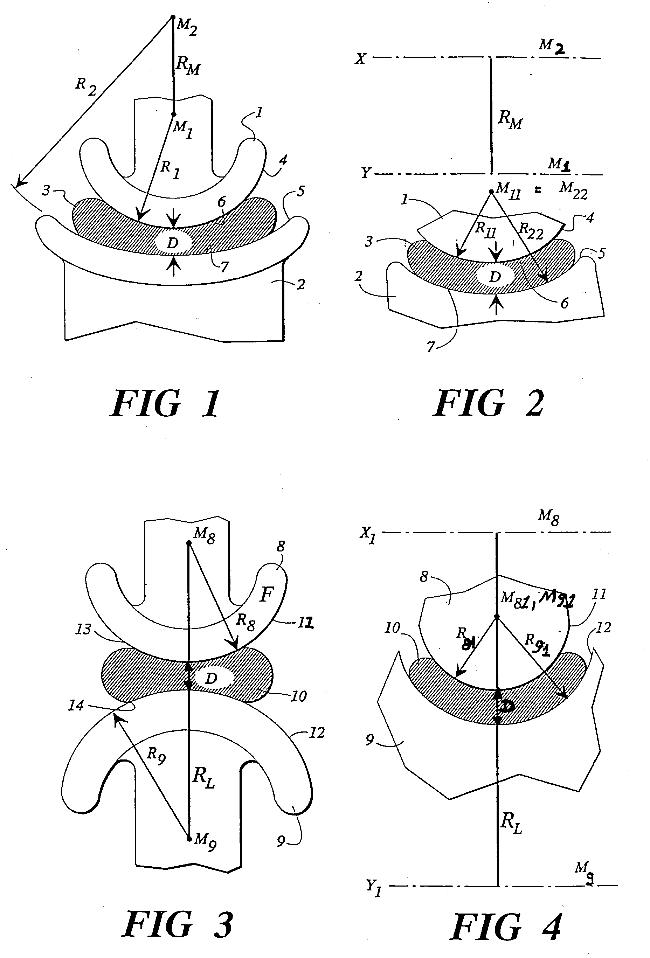

Ein menschliches Kniegelenk besteht aus zwei Gelenkteilen, und zwar dem medialen Gelenkteil und dem lateralen Gelenkteil. In Fig. 1 ist der Schnitt durch das künstliche Gelenk dargestellt, das als Ersatz für das mediale Gelenkteil dienen kann. Dieses Gelenk soll die natürliche Artikulation zwischen dem medialen femoralen (Oberschenkel) Gelenkkopf (Kondylus) und dem medialen tibialen (Schienbein) Gelenkkopf (Kondylus) ersetzen. Dieses erste Gelenk besteht aus zwei Gelenkteilen 1 und 2, wobei das Gelenkteil 1, erster Gelenkkopf, dem Femurteil (Oberschenkelteil) entsprechen kann, das starr mit dem Knochen des medialen femoralen Kondylus verbunden wird. Das Gelenkteil 2, erste Gelenkpfanne, entspricht dem Tibiateil (Schienbeinteil), der starr mit dem Knochen des tibialen Kondylus verbunden wird. Zwischen den beiden Gelenkteilen 1 und 2 befindet sich ein Druckverteilungskörper 3. Gemäß der vorliegenden Ausgestaltung ist nun vorgesehen, daß die Gelenkfläche 4 des Gelenkteils 1 und die Gelenkfläche 5 des Gelenkteils 2 jeweils als Flächensegmente eines toroidförmigen Körpers ausgebildet sind. Die den Gelenkflächen 4, 5 zugekehrten Gleitflächen 6, 7 des Diskus 3 sind entsprechend in Anpassung an die Gelenkflächen 4, 5 toroidförmig ausgestaltet. Wie sich aus Fig. 1 ergibt, weist das Gelenkteil 1 in der sagittalen Schnittebene, d.h. in der Längsebene, eine Funktionsfläche mit kreisbogenförmiger Schnittkontur auf, deren Rotationszentrum $M_1$ ist und wobei die kreisförmige Schnittkontur den Radius $R_1$ besitzt. Hierbei besitzt die derart gebildete Funktionsfläche eine konvexe Ausbildung. Das Gelenkteil 2 besitzt ebenfalls eine kreisförmige Schnittkontur mit dem Rotationszentrum $M_2$ und mit dem Radius $R_2$, so daß sich eine konkave Ausbildung der durch die kreisförmige Schnittkontur gebildeten Funktionsfläche ergibt. Hierbei ist eine derartige Anordnung gegeben, daß diese Rotationszentren $M_1$ und $M_2$ innerhalb des Gelenkteils mit der konvexen Schnittkontur liegen und die Gelenkachsenbahn der Rotationszentren einen Radius $R = R_2 - R_1 - D$ besitzen. Hierbei ist D die minimale Dicke des Diskus 3 auf der Verlängerung der Verbindungslinie der beiden Rotationszentren $M_1$ und $M_2$. Hierbei ist $R_2$ derart bemessen, daß die Summe aus $R_1$ und D kleiner ist als $R_2$. Somit stellt diese Anordnung eine überschlagene dimere Gelenkkette dar.

In Fig. 2 ist zu erkennen, daß auch in der Querebene, d.h. der Frontalebene, die Funktionsflächen der beiden Gelenkteile 1, 2 kreisförmige Schnittkonturen besitzen, wobei die kreisförmige Schnittkontur der Funktionsfläche des Gelenkteils 1 den Radius $R_{11}$ und den Mittelpunkt bzw. das Rotationszentrum $M_{11}$ besitzt und am Gelenkteil 2 die Funktionsfläche den Radius $R_{22}$ sowie den Mittelpunkt $M_{22}$. Auch hier ist wiederum der Druckverteilungskörper 3 zwischen den beiden Gelenkteilen 1, 2 zu erkennen mit seiner minimalen Diskusdicke D. Im dargestellten Ausführungsbeispiel fallen die Mittelpunkte $M_{11}$ und $M_{22}$ aufeinander. Die Rotationszentren $M_{11}$ und $M_{22}$ müssen nicht zusammenfallen. Das Rotationszentrum $M_{22}$ kann in Richtung des Oberschenkels und/oder seitlich nach innen (medial) oder außen (lateral) versetzt von dem Rotationszentrum $M_{11}$ liegen. Dies ist jedoch lediglich eine zweckmäßige Ausgestaltung. Weiterhin ist in Fig. 2 zu erkennen, wie die Rotationsachsen X, Y durch die Rotationszentren $M_2$ und $M_1$ in Fig. 1 innerhalb des frontalen Schnittes gemäß Fig. 2 verlaufen. Dabei ist zu erkennen, daß die Mittelpunkte $M_{11}$ und $M_{22}$ nicht mit den Rotationsachsen X, Y durch die Mittelpunkte $M_1$ bzw. $M_2$ zusammenfallen.

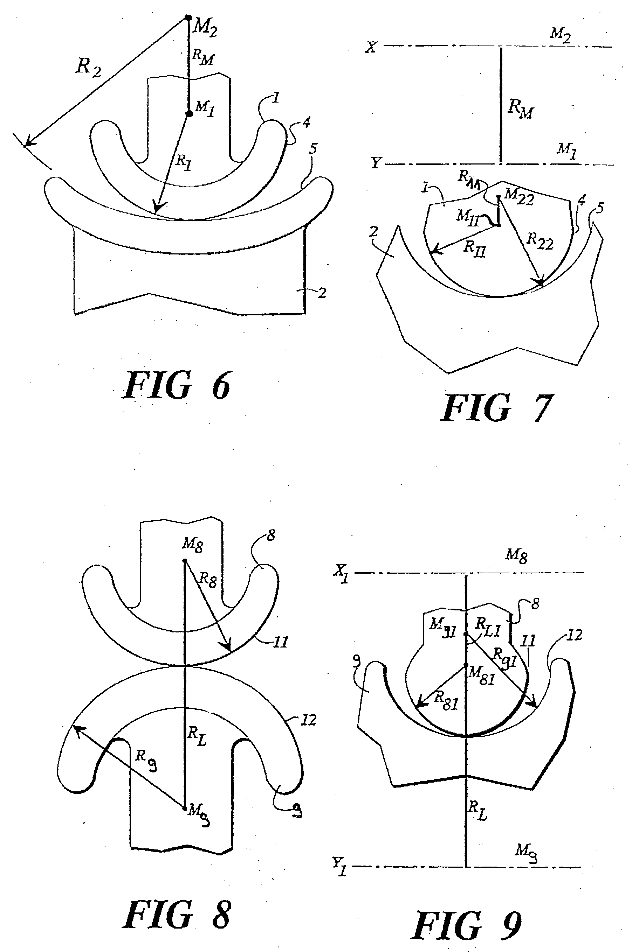

In den Fig. 6 und 7 ist ein dem Gelenk gemäß Fig. 1 und 2 entsprechendes Gelenk, jedoch ohne Druckverteilungskörper, dargestellt. Hierbei sind gleiche Teile jeweils mit denselben Bezugsziffern versehen. In bezug auf die Dimensionierung der geometrischen Beziehungen der jeweiligen Radien zueinander gilt die Bedingung D = 0. Geht man dabei davon aus, daß der Radius $R_M$ bei beiden Gelenken gemäß Fig. 1 und 2 bzw. Fig. 6 und 7 gleich groß ist, so wird man zweckmäßigerweise beim Gelenk gemäß den Fig. 6, 7 $R_1$ und $R_{11}$ gegenüber den entsprechenden Radien in den Fig. 1, 2 vergrößern. Diese Maßnahme ist deshalb zweckmäßig, da hierdurch die Berührungsfläche zwischen den Gelenkflächen 5 und 6 möglichst groß ist.

In Fig. 3 ist wiederum ein Schnitt durch die Längsebene bzw. in der sagittalen Ebene eines zweiten Gelenks dargestellt, das den lateralen Gelenkteil eines menschlichen Kniegelenkes bilden kann. Der laterale Gelenkteil ersetzt die natürliche Artikulation zwischen dem lateralen femoralen und lateralen tibialen Kondylus. Das Gelenk gemäß Fig. 3 besteht aus den Gelenkteilen 8, 9, und zwar einem zweiten Gelenkkopf 8 und einer zweiten Gelenkpfanne 9, zwischen denen ein Druckverteilungskörper 10 beweglich angeordnet ist. Das Gelenkteil 8 stellt dabei das Femurteil dar, das starr mit dem Knochen des lateralen femoralen Kondylus verbunden wird im menschlichen Körper, und das Gelenkteil 9 stellt das Tibiateil dar, das starr mit dem Knochen des lateralen tibialen Kondylus im menschlichen Körper verbunden wird. Die von den Gelenkteilen 8, 9 gebildeten Gelenkflächen 11, 12 sind als Flächensegmente eines toroidförmigen Körpers ausgebildet, so daß die den Gelenkflächen 11, 12 zugekehrten Gleitflächen 13, 14 des Diskus 10 ebenfalls in Anpassung an die Ausgestaltung der Gelenkflächen 11, 12 toroidförmig ausgestaltet sind. Das Gelenkteil 8 weist in Fig. 3 in seiner Längsebene ebenfalls eine Funktionsfläche mit einer kreisförmigen Schnittkontur auf, wobei diese kreisförmige Schnittkontur den Mittelpunkt bzw. das Rotationszentrum $M_8$ und den Radius $R_8$ besitzt, so daß sich ein konvexer Verlauf der Funktionsfläche ergibt. Das Gelenkteil 9 weist den Mittelpunkt $M_9$ bzw. das Rotationszentrum $M_9$ auf und besitzt den Radius $R_9$, so daß sich ebenfalls eine konvexe Form der kreisbogenförmigen Schnittkontur der Funktionsfläche ergibt. Wie dargestellt ist, liegen die Rotationszentren $M_8$ und $M_9$ jeweils im zugehörigen Gelenkteil 8 und 9. Die Gelenkachsenbahn der Rotationszentren $M_8$ und $M_9$ besitzt einen Radius $R_L = R_8 + R_9 + D$, wobei D die minimale Dicke des Diskus 10 auf der Verbindung zwischen den beiden Rotationszentren $M_8$ und $M_9$ ist.

In Fig. 4 ist der Schnitt gemäß der Frontalebene, Querebene, zu der Darstellung in Fig. 3 dargestellt. Hierbei ist zu erkennen, daß auch in dieser Schnittebene die Gelenkkörper 8, 9 jeweils kreisförmige Schnittkonturen ihrer Funktionsflächen besitzen. Der Gelenkkörper 8 weist dabei eine kreisförmige Schnittkontur mit dem Mittelpunkt $M_{81}$ und dem Radius $R_{81}$ auf, wobei eine konvexe Funktionsfläche gebildet wird. Der Gelenkkörper 9 besitzt den Mittelpunkt $M_{91}$ und den Radius $R_{91}$, wobei eine konkave Ausbildung der kreisförmigen Schnittkontur besteht. Hierbei liegen die Rotationszentren bzw. die Mittelpunkte $M_{81}$ und $M_{91}$ innerhalb des Gelenkteils 8 mit der konvexen Schnittkontur der Funktionsfläche, und die Gelenkachsenbahn der Rotationszentren $M_{81}$, $M_{91}$ ist im dargestellten Ausführungsbeispiel aufeinanderfallend angeordnet. Die

Rotationszentren $M_{81}$ und $M_{91}$ müssen ebenfalls nicht zusammenfallen. Das Rotationszentrum $M_{91}$ kann in Richtung auf den Oberschenkel und/oder seitlich nach innen (medial) oder außen (lateral) versetzt von dem Rotationszentrum $M_{81}$ liegen. Weiterhin ist zu erkennen, daß die Rotationszentren $M_{81}$ und $M_{91}$ nicht mit den Rotationsachsen $X_1$, $Y_1$ durch die Rotationszentren $M_8$ und $M_9$ zusammenfallen. Die kreisbogenförmigen Funktionsflächen in Fig. 3 stellen eine nicht überschlagene druckkraftschlüssige dimere Gelenkkette dar, wobei wegen der toroidförmigen Ausformung der Gelenkflächen keine Bewegungsfreiheit in der frontalen Ebene, siehe Fig. 4, gegeben ist.

Wesentlich für die einwandfreie Funktion der erfindungsgemäßen Gelenke gemäß den Fig. 1 bis 4 ist, daß die Druckverteilungskörper 3 bzw. 10 beweglich sind. Hierzu müßten die Gleitflächen 6, 7 zwischen den Druckverteilungskörpern und den Gelenkteilen 1, 2 bzw. 8, 9 eine möglichst geringe Reibung zu den Gelenkteilen besitzen, und zudem muß die Reibungskraft zwischen den anliegenden Flächen beidseitig des Druckverteilungskörpers jeweils gleich groß sein. Dies wird dadurch erreicht, daß die wirksamen Gleitflächen 6, 7 der Druckverteilungskörper 3, 10 zu den jeweils angrenzenden Gelenkteilen, das sind die Gelenkteile 1, 2 bzw. 8, 9, gleich groß ausgelegt werden. Die Größe der jeweiligen Kontaktfläche kann durch die Wahl der toroidalen Wulstradien sowie durch die Gestaltung der Randwülste der Druckverteilungskörper erreicht werden. Zudem sind die Gleitflächen 6, 7; 13, 14 hochglanzpoliert.

In den Fig. 8 und 9 ist ein dem Gelenk gemäß den Fig. 3 und 4 entsprechendes Gelenk jedoch ohne Druckverteilungskörper dargestellt. Hierbei sind gleiche Teile jeweils mit denselben Bezugsziffern versehen. Hinsichtlich der geometrischen Beziehungen der jeweiligen Radien zueinander gilt hier die Bedingung D = 0. Geht man hierbei davon aus, daß der Radius $R_L$ bei beiden Gelenken gemäß den Fig. 3, 4 und den Fig. 8, 9 gleich groß ist, so wird man zweckmäßigerweise $R_8$ und $R_{81}$ gemäß den Fig. 6 und 7 größer wählen als beim Gelenk gemäß Fig. 3, 4. Durch diese Maßnahme wird die Berührungsfläche zwischen den Gelenkflächen 11, 12 möglichst groß.

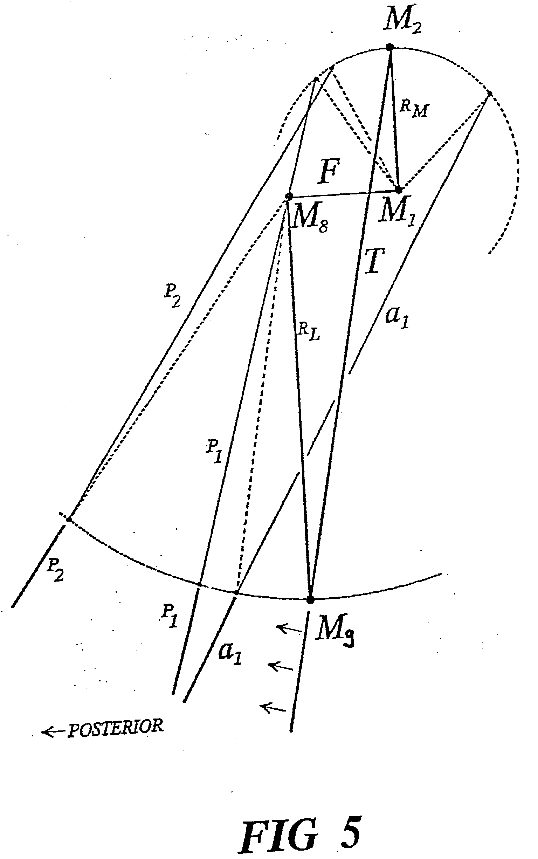

In Fig. 5 ist schematisch der erfindungsgemäße Aufbau eines rechten Knies im sagittalen Schnitt mit seitlicher Ansicht dargestellt, und zwar mittels des ersten Gelenks der Fig. 1, 2 bzw. Fig. 6, 7 und des zweiten Gelenks gemäß Fig. 3, 4 bzw. Fig. 8, 9. Hierbei sind die einzelnen Gelenkteile derart miteinander gekoppelt, daß der femorale erste und zweite Gelenkkopf 1, 8 und die tibiale erste und zweite Gelenkpfanne 2, 9 jeweils starr miteinander verbunden sind. Die beiden Gelenke aus den Gelenkteilen 1, 2 bzw. 8, 9 sind erfindungsgemäß so eingebaut, daß ihre vier Drehachsen X, Y und $X_1$ und $Y_1$ durch die Rotationszentren $M_1$ und $M_2$ bzw. $M_8$ und $M_9$ parallel zueinander in zwei parallelen Ebenen verlaufen und daß das zweite Gelenk aus den Gelenkteilen 8, 9 gegenüber dem ersten Gelenk aus den Gelenkteilen 1, 2 etwas nach posterior, d.h. nach hinten, versetzt ist. Ein derartiges Gelenkgebilde stellt ein Viergelenk dar, wobei das in Fig. 5 mit F gekennzeichnete Gelenkteil mit dem Femur, und das mit T dargestellte Gelenkteil mit der Tibia verbunden ist. Im Koordinatensystem des Femur ist F das Gestell, T die Koppel, $R_L$ und $R_M$ die rotierenden Glieder. Die Relativbewegung der Tibia gegenüber dem Femur stellt sich also als Bewegung der Koppel T dar. Da die Länge von T größer ist als die Summe aus $R_M + R_L$ kann sich die Achse $M_8$ aus der dick gezeichneten Initialstellung heraus nur nach posterior bewegen. Die Achse $M_2$ kann sich sowohl nach anterior als nach posterior bewegen. In beiden Fällen schwenkt aber die distale Verlängerung von T, der Unterschenkel, nach hinten. Beide Fälle stellen zwei mögliche Kniebeugebewegungen dar, wobei jede einzelne für sich zwangläufig abläuft. Bei der anterioren Bewegung der Drehachse $M_2$ bewegt sich diese nach Überschreiten der anterioren Totlage (RM und T bilden eine gerade Linie und fallen zusammen) weiter nach anterior. Die Tibia kann dann die dünn gezeichnete Stellung $a_1$ einnehmen. Bei der posterioren Bewegung von $M_2$ erreicht diese Achse in der posterioren Totlage ($R_L$ und T bilden eine gerade Linie und fallen zusammen, gezeichnete Lage $p_1$) ihre posteriorste Lage. Im Gefolge der weiteren Bewegung wandert $M_2$ dann in anteriore Richtung. Diese Wanderung geschieht so langsam, daß bei diesem weiteren Schwenken des Unterschenkels (Tibia T) die Drehachse $M_2$ an ihrem Ort zu verharren scheint (Lage $p_2$ der Tibia). In jedem Fall ($a_1$, $p_1$, $P_2$) ist der Unterschenkel nach hinten geschwenkt. Das künstliche Gelenk ist also so konstruiert, daß unter der Wirkung der kraftschlüssigen kompressiven Kräfte der Unterschenkel nur nach hinten schwenken kann.

Weiterhin ist es möglich, die minimale Stärke der Druckverteilungskörper auf Null zu reduzieren, so daß sich ein ringförmiger Ring-Druckverteilungskörper mit einer mittleren Öffnung ergibt.

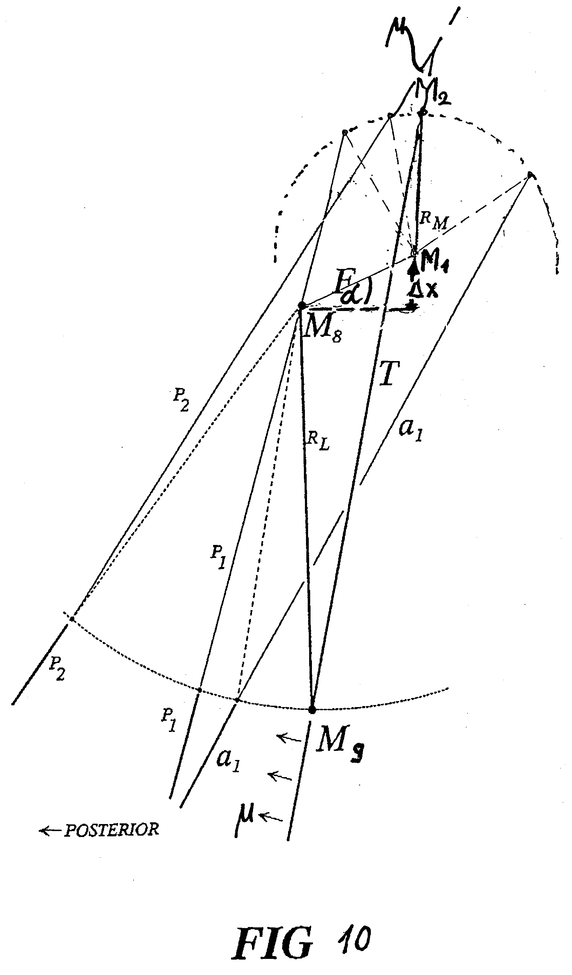

In Fig. 10 ist eine weitere Ausgestaltung eines erfindungsgemäßen Gelenks in der Darstellung gemäß Fig. 5 gezeigt. Diese Gelenkanordnung zum. Ersatz des menschlichen Kniegelenks zeigt, daß das erste Gelenk aus den Gelenkteilen 1, 2, das medial angeordnet ist, gegenüber dem lateral angeordneten Gelenk aus den Gelenkteilen 8, 9 in Richtung auf den Femur hinsichtlich seiner Gelenkmittelpunkte $M_1$, $M_2$ um das Maß $\Delta x$ versetzt ist. Durch die Größe dieses Versatzes kann der maximale Schwingwinkel $\mu_{max}$ des Gelenks, der dem maximalen Beugewinkel des menschlichen Knies entspricht, nach posterior beeinflußt werden. Hierbei besteht die Gesetzmäßigkeit, daß je größer der femorale Versatz $\Delta x$ ist, umso kleiner der maximale Schwingwinkel $\mu_{max}$ ausfällt. Vorzugsweise wird das femorale Versatzmaß $\Delta x$ derartig gewählt, daß die Verbindungsstrecke F von $M_8$ nach $M_1$ gegenüber der Horizontallinie einen Winkel $\alpha$ einschließt, der zwischen 0 und 45° liegt: $0 < \alpha < 45°$. Auch liegt es im Rahmen der vorliegenden Erfindung, die Gelenkteile 1, 2 bzw. 8, 9 des erfindungsgemäßen Gelenks derart anzuordnen, daß die Ebenen durch die Koppelglieder $R_L$ und $R_M$ räumlich zueinander geneigt verlaufen, woraus sich ein sphärischer Bewegungsablauf ergibt.

**Patentansprüche**

1. Künstliches Gelenk als Endoprothese für das menschliche Kniegelenk, bestehend aus einem ersten, aus einem ersten Gelenkkopf (1) und einer ersten Gelenkpfanne (2) gebildeten Gelenk und einem zweiten, aus einem zweiten Gelenkkopf (8) und einer zweiten Gelenkpfanne (9) gebildeten Gelenk, die zueinander derart parallel angeordnet sind, daß die jeweiligen Rotationsachsen (X, Y, $X_1$, $Y_1$) der beiden Gelenke parallel zueinander verlaufen, und daß das zweite Gelenk gegenüber dem ersten Gelenk in der Längsebene gesehen zurückversetzt ist sowie die Gelenkköpfe (1, 8) untereinander und die Gelenkpfannen (2, 9) untereinander starr verbindbar sind, wobei die Gelenkteile Funktionsflächen aufweisen, deren Krümmungsverhältnisse in ihrer Längsebene und in der hierzu um 90° versetzten Querebene entweder konvex-konvex und/oder konvex-konkav sind, und die Lenkgeometrie der Funktionsflächen zueinander in jeder der beiden Ebenen durch eine Gelenkkette mit zwei Gelenkachsen bestimmt ist, die durch die Rotationszentren ($M_1$, $M_2$, $M_{11}$, $M_{22}$; $M_8$, $M_9$, $M_{81}$, $M_{91}$) der Funktionsflächen mit den Radien ($R_1$, $R_2$, $R_{11}$, $R_{22}$; $R_8$, $R_9$, $R_{81}$, $R_{91}$) der jeweilig zugehörigen Schnittkonturen verlaufen, wobei eine femurseitige Verbindung (F) der Mittelpunkte ($M_1$, $M_8$) einem Gestell und eine tibiaseitige Verbindung (T) der Mittelpunkte ($M_2$, $M_9$) einer Koppel einer die vier Rotationsachsen (X, Y, $X_1$, $Y_1$) aufweisenden Viergelenkkette entsprechen.

2. Künstliches Gelenk nach Anspruch 1, **dadurch gekennzeichnet,** daß der zweite Gelenkteil das laterale Gelenkteil bildet und die Schnittkonturen des Gelenkkopfes (8) und der Gelenkpfanne (9) in der Längsebene konvex-konvex und in der Querebene konvex-konkav sind.

3. Künstliches Gelenk nach Anspruch 1 oder 2, **dadurch gekennzeichnet,** daß der Abstand (T) zwischen den tibiaseitigen Mittelpunkten ($M_2$, $M_9$) größer ist als die Summe aus $R_M + R_L$, wobei $R_M$ dem Abstand der Mittelpunkte ($M_1$, $M_2$) im ersten Teilgelenk (1, 2) und $R_L$ dem Abstand der Mittelpunkte ($M_8$, $M_9$) im zweiten Teilgelenk entspricht.

4. Künstliches Gelenk nach einem der Ansprüche 1 bis 3, **dadurch gekennzeichnet,** daß der Mittelpunkt ($M_8$) der femurseitigen Schnittkontur ($R_8$) des zweiten Teilgelenks (8, 9) gegenüber dem Mittelpunkt ($M_1$) der femurseitigen konvexen Schnittkontur ($R_1$)

des ersten Teilgelenks zusätzlich abwärts um einen Winkel $0 < \alpha < 45°$ versetzt ist.

EP 0 734 700 A2

**FIG 1**

**FIG 2**

**FIG 3**

**FIG 4**

6

**FIG 5**

**FIG 6**

**FIG 7**

**FIG 8**

**FIG 9**

*FIG 10*